# EUROPEAN PATENT SPECIFICATION

(11) **EP 1 933 818 B1**
(45) Date of publication and mention of the grant of the patent: **19.01.2011**
(21) Application number: 06814470.8
(22) Date of filing: 12.09.2006
(51) Int. Cl.: A61K 31/00, A61K 31/454, A61K 45/06, A61P 3/00

(54) **METHODS AND COMPOSITIONS USING IMMUNOMODULATORY COMPOUNDS FOR THE TREATMENT OF DISORDERS ASSOCIATED WITH LOW PLASMA LEPTIN LEVELS**
VERFAHREN UND ZUSAMMENSETZUNG, DIE IMMUNOMODULATORISCHE VERBINDUNGEN ZUR BEHANDLUNG VON MIT NIEDRIGEN PLASMALEPTIN-SPIEGELN IN ZUSAMMENHANG STEHENDEN STÖRUNGEN VERWENDEN
METHODES ET COMPOSITIONS UTILISANT DES COMPOSES IMMUNOMODULATEURS POUR LE TRAITEMENT DES TROUBLES ASSOCIES A DE FAIBLES TAUX DE LEPTINE DU PLASMA

(30) Priority: 12.09.2005 US 715582 P
(43) Date of publication of application: 25.06.2008
(73) Proprietor: CELGENE CORPORATION, Summit, NJ 07901 (US)
(72) Inventor: VERHELLE, Dominique, San Diego, CA 92130 (US); CHAN, Kyle, San Diego, CA 92130 (US); BRADY, Helen, San Diego, CA 92128 (US)
(74) Representative: Ritter, Thomas Kurt
(86) International application number: PCT/US2006/035378
(87) International publication number: WO 2007/033112

(56) References cited:
- WO-A-02/059106
- WO-A-02/072092
- WO-A-2005/105088
- WO-A2-03/034996
- WO-A2-2004/037199
- US-A- 5 635 517
- US-A1- 2004 087 546
- GABAY CEM ET AL: "Leptin directly induces the secretion of interleukin 1 receptor antagonist in human monocytes" JOURNAL OF CLINICAL ENDOCRINOLOGY AND METABOLISM, vol. 86, no. 2, February 2001 (2001-02), pages 783-791, XP002419250 ISSN: 0021-972X
- LIN HUI ZHI ET AL: "Metformin reverses fatty liver disease in obese, leptin-deficient mice" NATURE MEDICINE, vol. 6, no. 8, September 2000 (2000-09), pages 998-1003, XP002419251 ISSN: 1078-8956
- DATABASE WPI Week 200229 Derwent Publications Ltd., London, GB; AN 2002-241704 XP002419253 & WO 02/11725 A1 (SHIONOGI & CO LTD) 14 February 2002 (2002-02-14)

## Description

This invention relates to immunomodulatory compounds for use in treating, preventing and/or managing diseases or disorders associated with low plasma leptin levels. The invention also encompasses the use of specific combinations, or "cocktails," of immunomodulatory compounds, drugs and other therapies for treating such diseases or disorders. Also described herein are pharmaceutical compositions and dosing regimens.

### DISORDERS ASSOCIATED WITH HYPOLEPTINEMIC STATE

Leptin is a protein hormone predominantly secreted by adipocytes. It has been reported that leptin plays a significant role in regulating body weight, metabolic and endocrine functions, immunity, inflammation, and hematopoiesis. *See, e.g.,* Fantuzzi et al., Journal of Leukocyte Biology, 68: 437-446 (2000).

Leptin is also believed to play a role in skin repair and hair growth. It has been reported that systemic and topical application of recombinant leptin improves re-epithelialization of wounds in ob/ob mice, and accelerates normal wound-healing conditions in wild type mice. Frank et al., J. Invest. Clin., 106: 501-509 (2000) and Ring et al., Endocrinology, 141: 446-449 (2000). During skin repair, leptin acts as a mitogenic agent by inducing proliferation of keratinocytes. *See, e.g.,* Goren et al., Biochem. Biophys. Res. Comm., 303: 1080-1085 (2003) and Stallmeyer et al., J. Invest. Dermatol., 117: 98-105 (2001). Leptin has also been implicated in human hair biology. Iguchi et al., J. Invest. Dermatol., 117: 1349-1356 (2001).

Disorders associated with leptin deficiency often represent severe metabolic disorders. One example is lipodystrophy syndrome, which is characterized by reduction of glucose and triglyceride levels, and/or reduction of liver size. Insulin resistance and hypertriglyceridemia that characterize lipodystrophy have been reported to be refractory to treatments. *See, e.g.,* Garg, Am J. Med., 108: 143-152 (2000). Recently, it was reported that leptin therapy results in the amelioration of the major metabolic features from lipodystrophy syndrome. Oral et al., New England Journal of Medicine, 346: 570-578 (2002) and Peterson et al., JCI, 109: 1345-1350 (2002).

In anorexia nervosa, the chronic starvation induces depletion of fat stores, which is accompanied by alterations of circulating adipokines.Plasma leptin levels decrease, and adiponectin levels increase in anorexic state.In addition, anorexic patients suffer from endocrine and metabolic anomalies such as amenorrhea, delayed puberty, hypothyroidism, hypercorticosolism, and alterations in the growth hormone (GH) axis, bone metabolism and immune functions. Munoz et al., J. Pediatr. Endocrinol. Metab., suppl. 3: 478-480 (2004) and Brichard et al., Horm. Metab. Res., 35: 337-342 (2003). Leptin replacement therapy has been reported to result in increase in plasma thyroid hormone levels, age-dependent effects on gonadotropin secretion and pubertal development, and improvement in immunodeficiencies. Farooql et al., JCI, 110: 1093-11030 (2002). It was also reported that increase in serum leptin levels have favorable effects in long-term hemodialysis patients, resulting in better nutritional status and erythopoietin response. Hung et al., American Journal of Kidney Diseases, 45(6): 1073-1083 (2005).

Typically, these disorders have been treated using a combination of medications including, for example, insulin, oral hypoglycemic agents (*e.g.,* metformin and thiazolidinediones ("TZD")), and lipid lowering drugs such as fibrates and statins. However, known therapies are of limited value. For example, in the case of lipodystrophy, patients continue to have severe hyperglyceridemia, leading to recurrent attacks of acute pancreatitis, severe hyperglycemia and diabetic retinopathe and nephropathy potentially associated with it, and nonalcoholic steatohepatitis and potential cirrhosis.

In addition, although leptin replacement therapy is reported to have a much improved efficacy, it also has many problems. For example, the treatment can become painful and bothersome since the current leptin therapy involves daily intravenous administrations of recombinant leptin. Furthermore, by using recombinant leptin, the current leptin replacement therapy can only provide constant doses of leptin, which may differ significantly from the natural fluctuation of leptin levels in the body, and the gender-based difference of leptin levels. Therefore, a need exists for effective and convenient therapies for disorders associated with hypoleptinemic state.

### IMIDS™

A number of studies have been conducted with the aim of providing compounds that can safely and effectively be used to treat diseases associated with abnormal production of TNF-α. *See, e.g.,* Marriott, J.B., et al., Expert Opin. Biol. Ther. 1(4):1-8 (2001); G.W. Muller, et al., Journal of Medicinal Chemistry, 39(17): 3238-3240 (1996); and G.W. Muller, et al., Bioorganic & Medicinal Chemistry Letters, 8: 2669-2674 (1998). Some studies have focused on a group of compounds selected for their capacity to potently inhibit TNF-α production by LPS stimulated PBMC. L.G. Corral, et al., Ann. Rheum. Dis. 58:(Suppl I) 1107-1113 (1999). These compounds, which are referred to as IMiDs™ (Celgene Corporation) or Immunomodulatory Drugs, show not only potent inhibition of TNF-α but also marked inhibition of LPS induced monocyte IL1β and IL12 production. LPS induced IL6 is also inhibited by immunomodulatory compounds, *albeit* partially. These compounds are potent stimulators of LPS induced IL10. *Id*. Particular examples of IMiD™s include, but are not limited to, the substituted 2-(2,6-dioxopiperidin-3-yl) phthalimides and substituted 2-(2,6-dioxopiperidin-3-yl)-1-oxoisoindoles described in United States Patent Nos. 6,281,230 and 6,316,471, both to G.W. Muller, et al.

This invention encompasses immunomodulatory compounds, and pharmaceutically acceptable salts, solvates (*e.g*., hydrates), or stereoisomers, for use in treating, preventing and/or managing diseases or disorders associated with a hypoleptinemic state, *e.g.,* low plasma leptin levels.

The present invention relates to an immunomodulatory compound, or a pharmaceutically acceptable salt, solvate, or stereoisomer thereof for use in treating, managing or preventing a disorder associated with a hypoleptinemic state, or a symptom thereof, wherein the disorder is a metabolic or eating disorder, hypoleptinemia related neuroendocrine dysfunction, hypoleptinemia related immunodeficiency, hypothalamic amenorrhea, acromegaly, hypoleptinemia related infertility syndrome, skin damage, wound, long-term hemodialysis, or loss of hair.

According to one embodiment, the immunomodulatory compound is of formula (I): 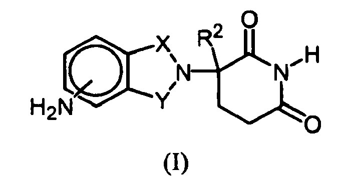 wherein one of X and Y is C=O, the other of X and Y is C=O or CH₂, and R² is hydrogen or methyl.

According to another embodiment, the immunomodulatory compound is of formula (II): 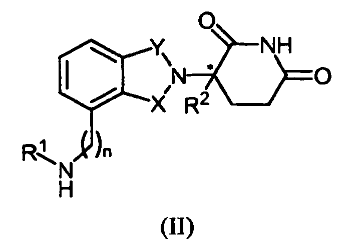 wherein one of X and Y is C=O and the other is CH₂ or C=O; R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵; R² is H, F, benzyl, (C₁-C₈)alkyl, (C₂-C₈)alkenyl, or (C₂-C₈)alkynyl; R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵; R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl; R⁵ is (C₁-C₈)alkyl, (C₂-C₈)alkenyl, (C₂-C₈)alkynyl, benzyl, aryl, or (C₂-C₅)heteroaryl; each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵, or the R⁶ groups join to form a heterocycloalkyl group; n is 0 or 1; and * represents a chiral-carbon center.

In some embodiments, the immunomodulatory compound is to be administered in combination with a therapy or agent conventionally used to treat, prevent and/or manage disorders associated with hypoleptinemic state.

**FIG. 1** is a schematic illustration of the generation of adipocytes from Mesenchymal Stem Cells ("MSCs").

**FIG. 2A** illustrates the effects of certain immunomodulatory compounds on triglyceride accumulation.

**FIG. 2B** illustrates the quantification of triglyceride accumulation in cells differentiated with DMSO or an immunomodulatory compound.

**FIG. 3A** illustrates the effects of 1-oxo-(2,6-dioxopiperidin-3-yl)-4-aminoisoindoline on leptin secretion in adipocytes.

**FIG. 3B** illustrates the effects of 1,3-dioxo-(2,6-dioxopiperidin-3-yl)-4-aminoisoindoline on leptin secretion in adipocytes.

**FIG. 3C** illustrates the effects of certain immunomodulatory compounds on leptin secretion in dexamethasone treated adipocytes.

**FIG. 4** illustrates the effects of certain immunomodulatory compounds on adiponectin secretion in adipocytes.

**FIG. 5** illustrates the mRNA levels of leptin and adiponectin in adipocytes treated with DMSO or an immunomodulatory compound.

**FIG. 6** illustrates the effects of certain immunomodulatory compounds on cell proliferation and viability.

A first embodiment of the invention encompasses an immunomodulatory compound of the invention, or a pharmaceutically acceptable salt, solvate (*e.g*., hydrate), stereoisomer, for use in treating, managing, and/or preventing a disease or disorder associated with a hypoleptinemic state.

In particular uses encompassed by this embodiment, the immunomodulatory compound is to be administered in combination with another drug ("second active agent") or other uses for treating, managing, and/or preventing a disorder associated with a hypoleptinemic state. Second active agents include small molecules and large molecules (*e.g*., proteins, antibodies, polynucleotides, and oligosaccharides), examples of which are provided herein, as well as stem cells.

The disorders associated with a hypoleptinemic state are metabolic and eating disorders such as, but not limited to, lipodystrophy syndrome and anorexia nervosa; hypoleptinemia related neuroendocrine dysfunctions; hypoleptinemia related immunodeficiencies; hypothalamic amenorrhea; acromegaly (pituitary adenoma); hypoleptinemia related infertility syndromes; skin damage; wounds; long-term hemodialysis; and loss of hair.

### DEFINITIONS

As used herein, and unless otherwise specified, the term "pharmaceutically acceptable salt" refers to salts prepared from pharmaceutically acceptable non-toxic acids, including inorganic acids and organic acids. Suitable non-toxic acids include inorganic and organic acids such as, acetic, alginic, anthranilic, benzenesulfonic, benzoic, camphorsulfonic, citric, ethenesulfonic, formic, fumaric, furoic, gluconic, glutamic, glucorenic, galacturonic, glycidic, hydrobromic, hydrochloric, isethionic, lactic, maleic, malic, mandelic, methanesulfonic, mucic, nitric, pamoic, pantothenic, phenylacetic, propionic, phosphoric, salicylic, stearic, succinic, sulfanilic, sulfuric, tartaric acid, p-toluenesulfonic. Suitable are hydrochloric, hydrobromic, phosphoric, and sulfuric acids.

As used herein, and unless otherwise specified, the term "solvate" means a compound of the present invention or a salt thereof, that further includes a stoichiometric or non-stoichiometric amount of solvent bound by non-covalent intermolecular forces. Where the solvent is water, the solvate is a hydrate.

As used herein, and unless otherwise specified, the term "stereoisomer" encompasses all enantiomerically/stereomerically pure and enantiomerically/stereomerically enriched compounds of this invention.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure when the compound contains 80%, 90%, or 95% or more of one stereoisomer and 20%, 10%, or 5% or less of the counter stereoisomer. In certain cases, a compound of the invention is considered optically active or stereomerically/enantiomerically pure (*i.e*., substantially the R-form or substantially the S-form) with respect to a chiral center when the compound is about 80% ee (enantiomeric excess) or greater, preferably, equal to or greater than 90% ee with respect to a particular chiral center, and more preferably 95% ee with respect to a particular chiral center.

As used herein, and unless otherwise indicated, the term "stereomerically enriched" or "enantiomerically enriched" also encompasses mixtures other than racemate such as mixtures of stereoisomers of compounds of this invention (*e.g*., R/S = 30/70, 35/65, 40/60, 45/55, 55/45, 60/40, 65/35 and 70/30).

As used herein, and unless otherwise specified, the terms "treat," "treating" and "treatment" contemplate an action that occurs while a patient is suffering from the specified disease or disorder, which reduces the severity of the disease or disorder, or retards or slows the progression of the disease or disorder.

As used herein, unless otherwise specified, the terms "prevent," "preventing" and "prevention" contemplate an action that occurs before a patient begins to suffer from the specified disease or disorder, which inhibits or reduces the severity of the disease or disorder.

As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

As used herein, and unless otherwise specified, the term "therapeutically effective amount" of a compound is an amount sufficient to provide a therapeutic benefit in the treatment or management of a disease or condition, or to delay or minimize one or more symptoms associated with the disease or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, the term "prophylactically effective amount" of a compound is an amount sufficient to prevent a disease or condition, or one or more symptoms associated with the disease or condition, or prevent its recurrence. The term "prophylactically effective amount" can encompass an amount that improves overall prophylaxis or enhances the prophylactic efficacy of another prophylactic agent.

### IMMUNOMODULATORY COMPOUNDS

In one embodiment, this invention relates to immunomodulatory compounds for use in treating, preventing, and/or managing diseases or disorders associated with hypoleptinemic state, *e.g.,* low plasma leptin levels. As used herein and unless otherwise indicated, the terms "immunomodulatory compounds" encompasses compounds of formula (I) and (II) that inhibit TNF-α, LPS induced monocyte IL 1β and IL12, and may also inhibit IL6 production. Specific immunomodulatory compounds of formula (I) and (II) are discussed below.

TNF-α is an inflammatory cytokine produced by macrophages and monocytes during acute inflammation. TNF-α is responsible for a diverse range of signaling events within cells. Without being limited by theory, one of the biological effects exerted by the immunomodulatory compounds of the invention is the reduction of TNF-α production. Immunomodulatory compounds of the invention may enhance the degradation of TNF-α mRNA.

Further, without being limited by theory, immunomodulatory compounds used in the invention may also be potent co-stimulators of T cells and increase cell proliferation dramatically in a dose dependent manner. Immunomodulatory compounds of the invention may also have a greater co-stimulatory effect on the CD8+ T cell subset than on the CD4+ T cell subset. In addition, the compounds preferably have anti-inflammatory properties, and efficiently co-stimulate T cells. Further, without being limited by a particular theory, immunomodulatory compounds used in the invention may be capable of acting both indirectly through cytokine activation and directly on Natural Killer ("NK") cells, and increase the NK cells' ability to produce beneficial cytokines such as, but not limited to, IFN-γ.

The immunomodulatory compounds include 1-oxo-and 1,3 dioxo-2-(2,6-dioxopiperidin-3-yl) isoindolines substituted with amino in the benzo ring as described in U.S. Patent no. 5,635,517 .

According to one embodiment of the invention, the immunomodulatory compound is of formula I:

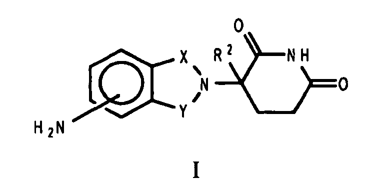

in which one of X and Y is C=O, the other of X and Y is C=O or CH₂, and R² is hydrogen or methyl. Specific immunomodulatory compounds include:

1-oxo-2-(2,6-dioxopiperidin-3-yl)-4-aminoisoindoline;

1-oxo-2-(2,6-dioxopiperidin-3-yl)-5-aminoisoindoline;

1-oxo-2-(2,6-dioxopiperidin-3-yl)-6-aminoisoindoline;

1-oxo-2-(2,6-dioxopiperidin-3-yl)-7-aminoisoindoline;

1,3-dioxo-2-(2,6-dioxopiperidin-3-yl)-4-aminoisoindoline;

1,3-dioxo-2-(2,6-dioxopiperidin-3-yl)-5-aminoisoindoline; and

1,3-dioxo-2-(3-methyl-2,6-dioxopiperidin-3-yl)-4-aminoisoindole.

Compounds representative of the invention are of the formulas:

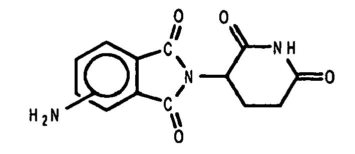

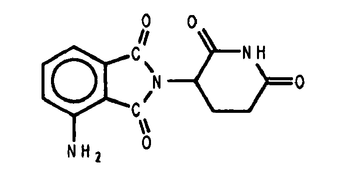

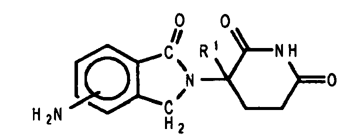

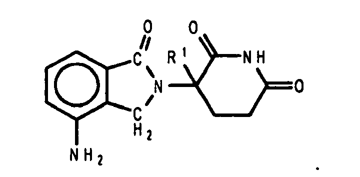

wherein R¹ is hydrogen or methyl. The use of enantiomerically pure forms (*e.g*. optically pure (R) or (S) enantiomers) of these compounds is described.

According to a further embodiment of the invention, the immunomodulatory compound is of formula II: 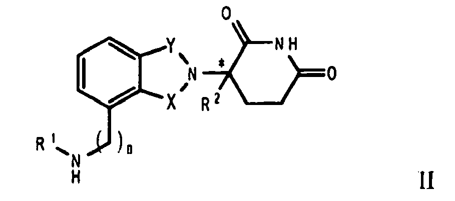 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R¹ is H, (C₁-C₈ )alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center.

In specific compounds of formula II, when n is 0 then R¹ is (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(O)OR⁴, (C₁-C₈)alkyl-N(R⁶)₂, (C₁-C₈)alkyl-OR⁵, (C₁-C₈)alkyl-C(O)OR⁵, C(S)NHR³, or (C₁-C₈)alkyl-O(CO)R⁵;
R² is H or (C₁-C₈)alkyl; and
R³ is (C₁-C₈)alkyl, (C₃-C₇)cycloalkyl, (C₂-C₈)alkenyl, (C₂-C₈)alkynyl, benzyl, aryl, (C₀-C₄)alkyl-(C₁ -C₆)heterocycloalkyl, (C₀-C₄)alkyl-(C₂-C₅)heteroaryl, (C₅-C₈)alkyl-N(R⁶)₂ ; (C₀-C₈)alkyl-NH-C(O)O-R⁵; (C₁-C₈)alkyl-OR⁵, (C₁-C₈)alkyl-C(O)OR⁵, (C₁-C₈)alkyl-O(CO)R⁵, or C(O)OR⁵; and the other variables have the same definitions.

In other specific compounds of formula II, R² is H or (C₁-C₄)alkyl.

In other specific compounds of formula II, R¹ is (C₁-C₈)alkyl or benzyl.

In other specific compounds of formula II, R¹ is H, (C₁-C₈)alkyl, benzyl, CH₂OCH₃, CH₂CH₂OCH₃, or

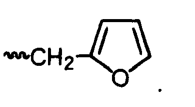

In another embodiment of the compounds of formula II, R¹ is 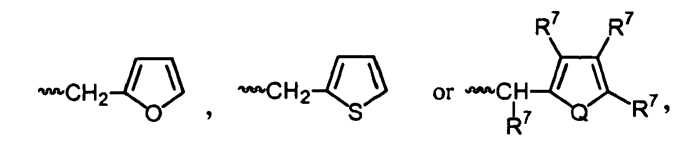 wherein Q is O or S, and each occurrence of R⁷ is independently H,(C₁-C₈)alkyl, (C₃-C₇)cycloalkyl, (C₂-C₈)alkenyl, (C₂-C₈)alkynyl, benzyl, aryl, halogen, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵, or adjacent occurrences of R⁷ can be taken together to form a bicyclic alkyl or aryl ring.

In other specific compounds of formula II, R¹ is C(O)R³.

In other specific compounds of formula II, R³ is (C0-C4)alkyl-(C2-C5)heteroaryl, (C1-C8)alkyl, aryl, or (C₀-C₄)alkyl-OR⁵.

In other specific compounds of formula II, heteroaryl is pyridyl, furyl, or thienyl.

In other specific compounds of formula II, R¹ is C(O)OR⁴.

In other specific compounds of formula II, the H of C(O)NHC(O) can be replaced with (C₁-C₄)alkyl, aryl, or benzyl.

Further examples of the compounds in this class include : [2-(2,6-dioxo-piperidin-3-yl)-1,3-dioxo-2,3-dihydro-1*H*-isoindol-4-ylmethyl]-amide; (2-(2,6-dioxo-piperidin-3-yl)-1,3-dioxo-2,3-dihydro-1*H*-isoindol-4-ylmethyl)-carbamic acid *tert-*butyl ester; 4-(aminomethyl)-2-(2,6-dioxo(3-piperidyl))-isoindoline-1,3-dione; *N*-(2-(2,6-dioxo-piperidin-3-yl)-1,3-dioxo-2,3-dihydro-1*H*-isoindol-4-ylmethyl)-acetamide; *N*-{(2-(2,6-dioxo(3-piperidyl)-1,3-dioxoisoindolin-4-yl)methyl}cyclopropyl-carboxamide; 2-chloro-*N*-{(2-(2,6-dioxo(3-piperidyl))-1,3-dioxoisoindolin-4-yl)methyl}acetamide; *N*-(2-(2,6-dioxo(3-piperidyl))-1,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; *N*-{(2-(2,6-dioxo(3-piperidyl))-1,3-dioxoisoindolin-4-yl)methyl}propanamide; *N*-{(2-(2,6-dioxo(3-piperidyl))-1,3-dioxoisoindolin-4-yl)methyl}-3-pyridylcarboxamide; *N*-{(2-(2,6-dioxo(3-piperidyl))-1,3-dioxoisoindolin-4-yl)methyl}heptanamide; *N*-{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; *N*-(2-(2,6-dioxo(3-piperidyl))-1,3-dioxoisoindolin-4-yl)pentanamide; *N*-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl] methyl}(butylamino)carboxamide; N-{[2-(2,6-dioxo(3-piperidyl))-1,3-dioxoisoindolin-4-yl] methyl}(octylamino)carboxamide; and N-{[2-(2,6-dioxo(3-piperidyl))-1,3-dioxoisoindolin-4-yl] methyl} (benzylamino)carboxamide.

The most preferred immunomodulatory compounds of the invention are 4-(amino)-2-(2,6-dioxo(3-piperidyl))-isoindoline-1,3-dione and 3-(4-amino-1-oxo-1,3-dihydroisoindol-2-yl)-piperidine-2,6-dione. The compounds can be obtained via standard, synthetic methods (*see e.g.,* United States Patent No. 5,635,517 ). The compounds are available from Celgene Corporation, Warren, NJ. 4-(Amino)-2-(2,6-dioxo(3-piperidyl))-isoindoline-1,3-dione has the following chemical structure:

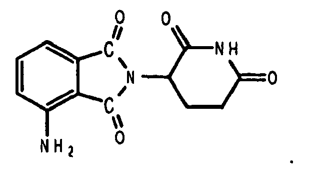

The compound 3-(4-amino-1-oxo-1,3-dihydro-isoindol-2-yl)-piperidine-2,6-dione has the following chemical structure:

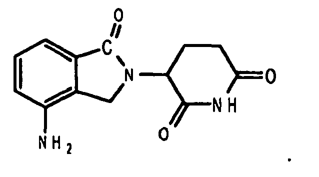

In another embodiment, specific immunomodulatory compounds of the invention encompass polymorphic forms of 3-(4-amino-1-oxo-1,3 dihydro-isoindol-2-yl)-piperidine-2,6-dione such as Form A, B, C, D, E, F, G and H, disclosed in U.S. publication no. 2005/0096351, published May 5, 2005. For example, Form A of 3-(4-amino-1-oxo-1,3 dihydro-isoindol-2-yl)-piperidine-2,6-dione is an unsolvated, crystalline material that can be obtained from non-aqueous solvent systems. Form A has an X-ray powder diffraction pattern comprising significant peaks at approximately 8, 14.5, 16, 17.5, 20.5, 24 and 26 degrees 2θ, and has a differential scanning calorimetry melting temperature maximum of about 270°C. Form A is weakly or not hygroscopic and appears to be the most thermodynamically stable anhydrous polymorph of 3-(4-amino-1-oxo-1,3 dihydro-isoindol-2-yl)-piperidine-2,6-dione discovered thus far.

Form B of 3-(4-amino-1-oxo-1,3 dihydro-isoindol-2-yl)-piperidine-2,6-dione is a hemihydrated, crystalline material that can be obtained from various solvent systems, including, hexane, toluene, and water. Form B has an X-ray powder diffraction pattern comprising significant peaks at approximately 16, 18, 22 and 27 degrees 2θ, and has endotherms from DSC curve of about 146 and 268°C, which are identified dehydration and melting by hot stage microscopy experiments. Interconversion studies show that Form B converts to Form E in aqueous solvent systems, and converts to other forms in acetone and other anhydrous systems.

Form C of 3-(4-amino-1-oxo-1,3 dihydro-isoindol-2-yl)-piperidine-2,6-dione is a hemisolvated crystalline material that can be obtained from solvents such as, acetone. Form C has an X-ray powder diffraction pattern comprising significant peaks at approximately 15.5 and 25 degrees 2θ, and has a differential scanning calorimetry melting temperature maximum of about 269°C. Form C is not hygroscopic below about 85% RH, but can convert to Form B at higher relative humidities.

Form D of 3-(4-amino-1-oxo-1,3 dihydro-isoindol-2-yl)-piperidine-2,6-dione is a crystalline, solvated polymorph prepared from a mixture of acetonitrile and water. Form D has an X-ray powder diffraction pattern comprising significant peaks at approximately 27 and 28 degrees 2θ, and has a differential scanning calorimetry melting temperature maximum of about 270°C. Form D is either weakly or not hygroscopic, but will typically convert to Form B when stressed at higher relative humidities.

Form E of 3-(4-amino-1-oxo-1,3 dihydro-isoindol-2-yl)-piperidine-2,6-dione is a dihydrated, crystalline material that can be obtained by slurrying 3-(4-amino-1-oxo-1,3 dihydro-isoindol-2-yl)-piperidine-2,6-dione in water and by a slow evaporation of 3-(4-amino-1-oxo-1,3 dihydro-isoindol-2-yl)-piperidine-2,6-dione in a solvent system with a ratio of about 9:1 acetone:water. Form E has an X-ray powder diffraction pattern comprising significant peaks at approximately 20, 24.5 and 29 degrees 2θ, and has a differential scanning calorimetry melting temperature maximum of about 269°C. Form E can convert to Form C in an acetone solvent system and to Form G in a THF solvent system. In aqueous solvent systems, Form E appears to be the most stable form. Desolvation experiments performed on Form E show that upon heating at about 125°C for about five minutes, Form E can convert to Form B. Upon heating at 175°C for about five minutes, Form B can convert to Form F.

Form F of 3-(4-amino-1-oxo-1,3 dihydro-isoindol-2-yl)-piperidine-2,6-dione is an unsolvated, crystalline material that can be obtained from the dehydration of Form E. Form F has an X-ray powder diffraction pattern comprising significant peaks at approximately 19, 19.5 and 25 degrees 2θ, and has a differential scanning calorimetry melting temperature maximum of about 269°C.

Form G of 3-(4-amino-1-oxo-1,3 dihydro-isoindol-2-yl)-piperidine-2,6-dione is an unsolvated, crystalline material that can be obtained from slurrying forms B and E in a solvent such as, tetrahydrofuran (THF). Form G has an X-ray powder diffraction pattern comprising significant peaks at approximately 21, 23 and 24.5 degrees 2θ, and has a differential scanning calorimetry melting temperature maximum of about 267°C.

Form H of 3-(4-amino-1-oxo-1,3 dihydro-isoindol-2-yl)-piperidine-2,6-dione is a partially hydrated (about 0.25 moles) crystalline material that can be obtained by exposing Form E to 0 % relative humidity. Form H has an X-ray powder diffraction pattern comprising significant peaks at approximately 15, 26 and 31 degrees 2θ, and has a differential scanning calorimetry melting temperature maximum of about 269°C.

Compounds of the invention can either be commercially purchased or prepared according to the methods described in the patents or patent publications disclosed herein. Further, optically pure compounds can be asymmetrically synthesized or resolved using known resolving agents or chiral columns as well as other standard synthetic organic chemistry techniques.

Various immunomodulatory compounds of the invention contain one or more chiral centers, and can exist as racemic mixtures of enantiomers or mixtures of diastereomers. This invention encompasses the use of stereomerically pure forms of such compounds, as well as the use of mixtures of those forms. For example, mixtures comprising equal or unequal amounts of the enantiomers of a particular immunomodulatory compounds of the invention may be used in methods and compositions of the invention. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

### SECOND ACTIVE AGENTS

Immunomodulatory compounds of the invention can be combined with other pharmacologically active compounds ("second active agents") in uses of the invention. The invention encompasses certain combinations that may be synergistic in the treatment, prevention and/or management of disorders associated with a hypoleptinemic state. The Immunomodulatory compounds can also work to alleviate adverse effects associated with certain second active agents.

This invention also encompasses the use of native, naturally occurring, and recombinant proteins.

The invention further encompasses mutants and derivatives (*e.g*., modified forms) of naturally occurring proteins that exhibit, *in vivo,* at least some of the pharmacological activity of the proteins upon which they are based. Examples of mutants include , proteins that have one or more amino acid residues that differ from the corresponding residues in the naturally occurring forms of the proteins. Also encompassed by the term "mutants" are proteins that lack carbohydrate moieties normally present in their naturally occurring forms (*e.g*., nonglycosylated forms). Examples of derivatives include, pegylated derivatives and fusion proteins, such as proteins formed by fusing IgG1 or IgG3 to the protein or active portion of the protein of interest. *See, e.g.,* Penichet, M.L. and Morrison, S.L., J. Immunol. Methods 248:91-101 (2001).

In one embodiment of the invention, the large molecule active agent reduces, eliminates, or prevents an adverse effect associated with the administration of the immunomodulatory compound. Depending on the particular immunomodulatory compound and the disease or disorder being treated, adverse effects can include, drowsiness and somnolence, dizziness and orthostatic hypotension, neutropenia, infections that result from neutropenia, increased HIV-viral load, bradycardia, Stevens-Johnson Syndrome and toxic epidermal necrolysis, and seizures (*e.g*., grand mal convulsions).

Second active agents that are small molecules can also be used to alleviate adverse effects associated with the administration of the immunomodulatory compound. However, like some large molecules, many are believed to be capable of providing a synergistic effect when administered with (*e.g*., before, after or simultaneously) the immunomodulatory compound.

Specific second active agents include: antibiotics such as, chloramphenicol, tetracycline, chlortetracycline, sulfasalazine, ampicillin, penicillin, and trimethoprim-sulfamethoxazole; dexamethasone; insulin; hypoglycemic agents such as, metformin and thiazolidinediones ("TZD"); lipid lowering agents such as, fibrates and statins; fluoxetine; clomiphene citrate; gonadotropins; GnRH; corticosteroids such as, betamethasone, clobetasol, amcinonide, desoximethasone, diflorasone, fluocinonide, halcinonide, mometasone, amcinonide, fluticasone, triamcinolone, fluocinolone, flurandrenolide, hydrocortisone, alclometasone, desonide, flumethasone, and pramoxine; minoxidil; finasteride; amylin and synthetic analogues thereof such as, pramlintide (symlin); and clomipramine.

In another embodiment, this invention can be used in combination with other methods used for the treatment, prevention, and/or management of a disorder associated with hypoleptinemic state. Examples of other methods include, but not limited to, surgeries and psychological therapies.

Specific uses of the invention comprise administering the immunomodulatory compound of the invention, or a pharmaceutically acceptable salt, solvate, or stereoisomer, in combination with one or more second active agents or other therapies. Examples of immunomodulatory compounds and second active agents and other therapies are disclosed herein.

Administration of the immunomodulatory compounds and the second active agents to a patient can occur simultaneously or sequentially by the same or different routes of administration. The suitability of a particular route of administration employed for a particular active agent will depend on the active agent itself (*e.g*., whether it can be administered orally without decomposing prior to entering the blood stream) and the disease being treated. A particular route of administration for an immunomodulatory compound of the invention is oral. Particular routes of administration for the second active agents or ingredients of the invention are known to those of ordinary skill in the art. *See, e.g.,* The Merck Manual, 1023-1041 (17th ed., 1999).

The amount of second active agent administered can be determined based on the specific agent used, the type of disease being treated or managed, the severity and stage of disease, and the amount(s) of immunomodulatory compounds of the invention and any optional additional active agents concurrently administered to the patient. Those of ordinary skill in the art can determine the specific amounts according to conventional procedures known in the art. In the beginning, one can start from the amount of the second active agent that is conventionally used in the therapies, and adjust the amount according to the factors described above. *See, e.g.,* Physician's Desk Reference (60th Ed., 2006).

In one embodiment of the invention, the second active agent is to be administered intravenously or subcutaneously and once or twice daily in an amount of from about 1 to about 1000 mg, from about 5 to about 500 mg, from about 10 to about 350 mg, or from about 50 to about 200 mg. The specific amount of the second active agent will depend on the specific agent used, the type of disease being treated or managed, the severity and stage of disease, and the amount(s) of immunomodulatory compounds of the invention and any optional additional active agents concurrently administered to the patient.

In one embodiment, an immunomodulatory compound can be administered in an amount of from about 0.1 to about 150 mg, and preferably from about 1 to about 25 mg, more preferably from about 2 to about 10 mg orally and daily alone, or in combination with a second active agent disclosed herein, prior to, during, or after the use of conventional therapy.

### USES FOR TREATMENTS AND PREVENTION

This invention encompasses uses for treating, preventing and/or managing various diseases and/or disorders associated with low levels of plasma leptin (hypoleptinemic state). As used herein, and unless otherwise specified, the term "diseases or disorders associated with hypoleptinemic state" means the diseases or disorders that are characterized by, or accompanied with, plasma leptin levels lower than those without such diseases or disorders. The term also encompasses diseases or disorders that respond favorably to the modulation (*e.g*., increase) in the plasma leptin levels.

Such disorders are metabolic and eating disorders such as, but not limited to, lipodystrophy syndrome and anorexia nervosa; hypoleptinemia related neuroendocrine dysfunctions; hypoleptinemia related immunodeficiencies; hypothalamic amenorrhea; acromegaly (pituitary adenoma); hypoleptinemia related infertility syndromes; skin damges; wounds; long-term hemodialysis; and loss of hair.

Further described are uses for treating, preventing, and/or managing metabolic disorders such as, obesity and diabetes comprising administration of an immunomodulatory compound, alone or in combination with another agent or therapy.

This invention also encompasses uses for the treatment, prevention and/or management of symptoms associated with these disorders. For example, this invention encompasses uses for treating, preventing, and/or managing the symptoms such as, neuroendocrine defects, immunodeficiences, high glucose and triglyceride levels, and enlarged liver, which are commonly associated with lipodystrophy. This invention also encompasses uses for treating, preventing, and/or managing the symptoms such as, endocrine and metabolic anomalies (*e.g*., amenorrhea, delayed puberty, hypothyroidism, hypercorticosolism, and alterations in growth hormone axis, circulating adipokines, bone metabolism, and immune functions).

According to a use of this invention one or more immunomodulatory compounds of the invention, or a pharmaceutically acceptable salt, solvate, or stereoisomer, are to be administered to a patient (*e.g*., a human) suffering, or likely to suffer, from a disorder associated with hypoleptinemic state.

In one embodiment of the invention, an immunomodulatory compound of the invention can be administered orally and in single or divided daily doses in an amount of from about 0.10 to about 150 mg/day. In other embodiments, an immunomodulatory compound is to be administered in an amount of from about 0.1 to about 1 mg per day, from about 1 to about 25 mg per day, from about 10 mg to about 25 mg per day, about 50 mg per day, or amount of about 25 mg per day. In other embodiments, an immunomodulatory compound is to be administered from about 0.1 to about 5 mg every other day or from about 10 to about 50 mg every other day. In other embodiments, the immunomodulatory compound is 4-(amino)-2-(2,6-dioxo(3-piperidyl))-isoindoline-1,3-dione or 3-(4-amino-1-oxo-1,3-dihydro-isoindol-2-yl)-piperidine-2,6-dione.

### CYCLING THERAPY

In certain embodiments, the prophylactic or therapeutic agents of the invention are to be cyclically administered to a patient. Cycling therapy involves the administration of an active agent for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

Consequently, in one specific embodiment of the invention, an immunomodulatory compound of the invention is to be administered daily in a single or divided doses in a four to six week cycle with a rest period of about a week or two weeks. The invention further allows the frequency, number, and length of dosing cycles to be increased. Thus, another specific embodiment of the invention encompasses uses for the administration of an immunomodulatory compound of the invention for more cycles than are typical when it is administered alone. In yet another specific embodiment of the invention, an immunomodulatory compound of the invention is to be administered for a greater number of cycles that would typically cause dose-limiting toxicity in a patient to whom a second active ingredient is not also being administered.

In one embodiment, an immunomodulatory compound of the invention is to be administered daily and continuously for three or four weeks at a dose of from about 0.1 to about 150 mg/d followed by a break of one or two weeks. In one embodiment, 4-(amino)-2-(2,6-dioxo(3-piperidyl))-isoindoline-1,3-dione is to be administered daily and continuously at an initial dose of 0.1 to 5 mg/d with dose escalation (every week) by 1 to 10 mg/d to a maximum dose of 50 mg/d for as long as therapy is tolerated. In another embodiment, 3-(4-amino-1-oxo-1,3-dihydro-isoindol-2-yl)-piperidine-2,6-dione is to be administered in an amount of about 1, 5, 10, or 25 mg/day for three to four weeks, followed by one week or two weeks of rest in a four or six week cycle. In another embodiment, 3-(4-amino-1-oxo-1,3-dihydro-isoindol-2-yl)-piperidine-2,6-dione is to be administered in an amount of about 10 mg/day for three to four weeks, followed by one week or two weeks of rest in a four or six week cycle.

In one embodiment of the invention, an immunomodulatory compound of the invention and a second active ingredient are to be administered orally, with administration of an immunomodulatory compound of the invention occurring 30 to 60 minutes prior to a second active ingredient, during a cycle of four to six weeks. In another embodiment of the invention, the combination of an immunomodulatory compound of the invention and a second active ingredient is to be administered by intravenous infusion over about 90 minutes every cycle. In one specific embodiment, one cycle comprises the administration of from about 1 to about 25 mg/day of 3-(4-amino-1-oxo-1,3-dihydro-isoindol-2-yl)-piperidine -2,6-dione and from about 50 to about 200 mg/m²/day of a second active ingredient daily for three to four weeks and then one or two weeks of rest. In another specific embodiment, each cycle comprises the administration of from about 5 to about 10 mg/day of 4-(amino)-2-(2,6-dioxo(3-piperidyl))-isoindoline-1,3-dione and from about 50 to about 200 mg/m²/day of a second active ingredient for 3 to 4 weeks followed by one or two weeks of rest. Typically, the number of cycles during which the combinatorial treatment is administered to a patient will be from about one to about 24 cycles, more typically from about two to about 16 cycles, and even more typically from about four to about three cycles.

### PHARMACEUTICAL COMPOSITIONS AND DOSAGE FORMS

Pharmaceutical compositions can be used in the preparation of individual, single unit dosage forms. Pharmaceutical compositions and dosage forms which can be used in the invention may comprise an immunomodulatory compound of the invention, or a pharmaceutically acceptable salt, solvate, or stereoisomer, and a second active agent. Pharmaceutical compositions and dosage forms can further comprise one or more excipients.

Pharmaceutical compositions and dosage forms can also comprise one or more additional active ingredients. Consequently, pharmaceutical compositions and dosage forms comprise the active ingredients disclosed herein (*e.g*., an immunomodulatory compound and a second active agent). Examples of optional second, or additional, active ingredients are disclosed herein.

Single unit dosage forms are suitable for oral, mucosal (*e.g*., nasal, sublingual, vaginal, buccal, or rectal), parenteral (*e.g*., subcutaneous, intravenous, bolus injection, intramuscular, or intraarterial), topical (*e.g*., eye drops or other ophthalmic preparations), transdermal or transcutaneous administration to a patient. Examples of dosage forms include: tablets; caplets; capsules, such as soft elastic gelatin capsules; cachets; troches; lozenges; dispersions; suppositories; powders; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions, or a water-in-oil liquid emulsions), solutions, and elixirs; liquid dosage forms suitable for parenteral administration to a patient; eye drops or other ophthalmic preparations suitable for topical administration; and sterile solids (*e.g*., crystalline or amorphous solids) that can be reconstituted to provide liquid dosage forms suitable for parenteral administration to a patient.

The composition, shape, and type of dosage forms will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms will vary from one another will be readily apparent to those skilled in the art. *See generally ,* Remington: The Science and Practice of Pharmacy, 20th Ed. (2000)..

Typical pharmaceutical compositions and dosage forms comprise one or more excipients. Suitable excipients are well known to those skilled in the art of pharmacy, and examples of suitable excipients are provided herein. Whether a particular excipient is suitable for incorporation into a pharmaceutical composition or dosage form depends on a variety of factors well known in the art including, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, pharmaceutical compositions and dosage forms can contain little, if any, lactose other mono- or di-saccharides. As used herein, the term "lactose-free" means that the amount of lactose present, if any, is insufficient to substantially increase the degradation rate of an active ingredient.

Lactose-free compositions of the invention can comprise excipients that are well known in the art and are listed, for example, in the *U.S. Pharmacopeia* (USP) 25-NF20 (2002). In general, lactose-free compositions comprise active ingredients, a binder/filler, and a lubricant in pharmaceutically compatible and pharmaceutically acceptable amounts. Particular lactose-free dosage forms comprise active ingredients, microcrystalline cellulose, pre-gelatinized starch, and magnesium stearate.

Further useful in the invention are anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include hermetically sealed foils, plastics, unit dose containers (*e.g*., vials), blister packs, and strip packs.

Further useful in the invention are pharmaceutical compositions and dosage forms that comprise one or more compounds that reduce the rate by which an active ingredient will decompose. Such compounds, which are referred to herein as "stabilizers," include antioxidants such as ascorbic acid, pH buffers, or salt buffers.

Like the amounts and types of excipients, the amounts and specific types of active ingredients in a dosage form may differ depending on factors such as, the route by which it is to be administered to patients. However, typical dosage forms comprise an immunomodulatory compound of the invention or a pharmaceutically acceptable salt, solvate, or stereoisomer, in an amount of from about 0.10 to about 150 mg. Typical dosage forms comprise an immunomodulatory compound of the invention or a pharmaceutically acceptable salt, solvate, stereoisomer in an amount of about 0.1, 1, 2, 5, 7.5, 10, 12.5, 15, 17.5, 20, 25, 50, 100, 150 or 200 mg. In a particular embodiment, a dosage form comprises 4-(amino)-2-(2,6-dioxo(3-piperidyl))-isoindoline-1,3-dione in an amount of about 1, 2, 5, 10, 25 or 50 mg. In a specific embodiment, a dosage form comprises 3-(4-amino-1-oxo-1,3-dihydro-isoindol-2-yl)-piperidine-2,6-dione in an amount of about 5, 10, 25 or 50 mg. Typical dosage forms comprise the second active ingredient in an amount of 1 to about 1000 mg, from about 5 to about 500 mg, from about 10 to about 350 mg, or from about 50 to about 200 mg. Of course, the specific amount of the agent will depend on the specific agent used, the type of disease or disorder being treated or managed, and the amount(s) of an immunomodulatory compound of the invention and any optional additional active agents concurrently administered to the patient.

### ORAL DOSAGE FORMS

Pharmaceutical compositions useful in the invention that are suitable for oral administration can be presented as discrete dosage forms, such as tablets (*e.g*., chewable tablets), caplets, capsules, and liquids (*e.g.,* flavored syrups). Such dosage forms contain predetermined amounts of active ingredients, and may be prepared by methods of pharmacy well known to those skilled in the art. *See generally ,* Remington: The Science and Practice of Pharmacy, 20th Ed. (2000).

Typical oral dosage forms are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, water, glycols, oils, alcohols, flavoring agents, preservatives, and coloring agents. Examples of excipients suitable for use in solid oral dosage forms (*e.g*., powders, tablets, capsules, and caplets) include, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, binders, fillers, disintegrants, and lubricants. Binders suitable for use in pharmaceutical compositions and dosage forms include, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g*., Nos. 2208, 2906, 2910), microcrystalline cellulose, and mixtures thereof.

Suitable forms of microcrystalline cellulose include, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Examples of fillers suitable for use in the pharmaceutical compositions and dosage forms disclosed herein include, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the invention include, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms include, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

A particular solid oral dosage form comprises an immunomodulatory compound of the invention, anhydrous lactose, microcrystalline cellulose, polyvinylpyrrolidone, stearic acid, colloidal anhydrous silica, and gelatin.

### DELAYED RELEASE DOSAGE FORMS

Active ingredients of the invention can be administered by controlled release means or by delivery devices that are well known to those of ordinary skill in the art. Examples include, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566 .
Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. Single unit dosage forms suitable for oral administration such as, tablets, capsules, gelcaps, and caplets that are adapted for controlled-release, can be used in the invention.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### PARENTERAL DOSAGE FORMS

Parenteral dosage forms can be administered to patients by various routes including, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include : Water for Injection USP; aqueous vehicles such as, Sodium Chloride Injection, Ringer's Injection, Dextrose Injection, Dextrose and Sodium Chloride Injection, and Lactated Ringer's Injection; water-miscible vehicles such as, ethyl alcohol, polyethylene glycol, and polypropylene glycol; and non-aqueous vehicles such as, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms. For example, cyclodextrin and its derivatives can be used to increase the solubility of an immunomodulatory compound of the invention and its derivatives. *See, e.g.,* U.S. Patent No. 5,134,127 .

### TOPICAL AND MUCOSAL DOSAGE FORMS

Topical and mucosal dosage forms useful in the invention include, sprays, aerosols, solutions, emulsions, suspensions, eye drops or other ophthalmic preparations, or other forms known to one of skill in the art. *See e.g.,* Remington: The Science and Practice of Pharmacy, 20th Ed. (2000); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels.

Suitable excipients (*e.g*., carriers and diluents) and other materials that can be used to provide topical and mucosal dosage forms are well known to those skilled in the pharmaceutical arts, and depend on the particular tissue to which a given pharmaceutical composition or dosage form will be applied. With that fact in mind, typical excipients include, water, acetone, ethanol, ethylene glycol, propylene glycol, butane-1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. *See e.g.,* Remington: The Science and Practice of Pharmacy, 20th Ed. (2000).

The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### KITS

Typically, active ingredients of the invention are preferably not administered to a patient at the same time or by the same route of administration. This invention enables the use of kits which, when used by the medical practitioner, can simplify the administration of appropriate amounts of active ingredients to a patient.

A typical kit comprises a dosage form of an immunomodulatory compound of the invention, or a pharmaceutically acceptable salt, solvate, or stereoisomer. Kits can further comprise additional active ingredients. Examples of the additional active ingredients include, those disclosed herein.

Kits can further comprise devices that are used to administer the active ingredients. Examples of such devices include, syringes, drip bags, patches, and inhalers.

Kits can further comprise cells or blood for transplantation as well as pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include: Water for Injection USP; aqueous vehicles such as, Sodium Chloride Injection, Ringer's Injection, Dextrose Injection, Dextrose and Sodium Chloride Injection, and Lactated Ringer's Injection; water-miscible vehicles such as, ethyl alcohol, polyethylene glycol, and polypropylene glycol; and non-aqueous vehicles such as, corn oil, cottonseed oil, peanut oil, sesame oil, ethyl oleate, isopropyl myristate, and benzyl benzoate.

### EXAMPLES

Certain embodiments of the invention are illustrated by the following examples.

### 1. EXPERIMENTAL PROCEDURES

### 1.1 GENERATION OF ADIPOCYTE FROM MESENCHYMAL STEM CELL

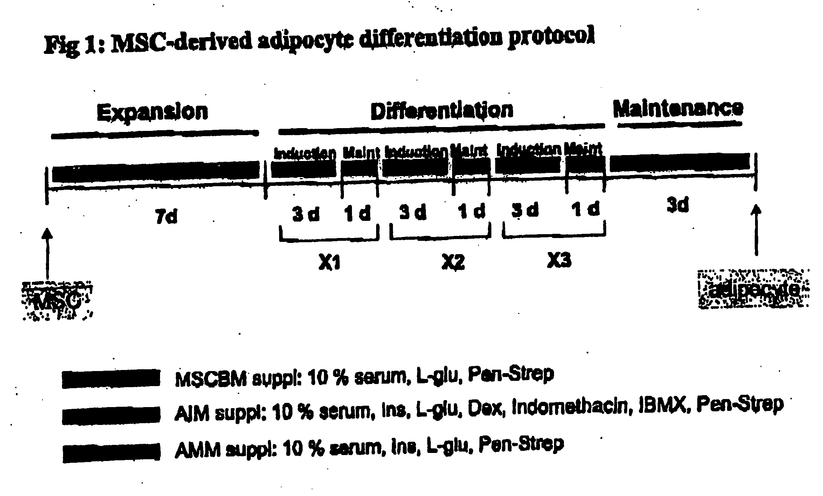

Human Mesenchymal Stem Cell (MSC) and adipocyte differentiation media were obtained from Cambrex (Walkersville, MD). MSCs were seeded in 24 well plates at 40,000 cells per well in MSC Basic Medium (MSCBM), supplemented with 10% serum and expanded for 7 days. At 100% of confluence, three cycles of induction/maintenance stimulated adipogetic differentiation. Each cycle consisted feeding MSCs with Adipogenic Induction Medium (AIM) for 3 days, followed by 1 day of culture in Adipogenic Maintenance Medium (AMM). AIM and AMM contained 10% serum, Pen-Sterep and L-Glu, supplemented with insulin, dexamethasone, indomethacin and IBMX, or insulin only. After 3 complete cycles of induction/maintenance, MSCs are cultured for 3 more days in AMM and analyzed. A schematic illustration of the procedures is shown in FIG. 1.

To study the effects of the immunomodulatory compounds on adipocyte differentiation, MSCs were differentiated in the presence of DMSO or varying concentrations of the immunomodulatory compound. DMSO and immunomodulatory compounds were added to the medium during the differentiation and maintenance steps. Phenotypic characterization of the cells for adipocyte markers, such as triglyceride content and adipokine secretion, was performed.

### 1.2 PROTEIN SECRETION

Leptin and adiponectin were measured using the quantikine human leptin and human adiponectin immunoassay kits (R&D Systems, Minneapolis, MN). To measure leptin secretion, 100 µl of medium was collected from the cell plates, diluted 2 or 5 times depending on the collecting time during the differentiation process, and analyzed following the manufacturer's protocols. Results were corrected for the dilution factor. For adiponectin secretion, 50 µl of medium was collected and directly analyzed.

### 1.3 OIL RED STAINING

Fat content in adipocyte was quantified by Oil Red O staining. Cells were washed with PBS and fixed with 3.7% formaldehyde for 15 minutes. Lipid vacuoles were stained with 0.5% Oil Red O in 60% isopropanol for 30 minutes at 37°C. Cells were then washed three times with water and Oil Red O content was dissolved into 100% propanol. The optical density of the solution was measured at 510 nm.

### 1.4 MSC VIABILITY AND PROLIFERATION

MSC viability was assayed using cell proliferation kit II (XTT) (Roche, Indianapolis, IN). MSC proliferation was assayed using 5-bromo-2'-deoxy-uridine labeling and detection kit III (BrdU) (Roche, Indianapolis, IN). MSCs have been seeded in 96 well plates and expanded for 2 days in presence of DMSO or varying concentrations of an immunomodulatory compound. XTT and BrdU were added to the cells for 20 hours. The viability and proliferation of MSCs were assayed following manufacturer's instructions.

### 1.5 NORTHERN BLOT

RNAs from MSC-derived adipocytes were isolated with RNeasy lipid tissue kit (Qiagen, Valencia, CA). Ten micrograms of total RNA were separated by electrophoresis on 1.2% agarose containing formaldehyde and transferred on a Hybond-XL membrane (Amersham Biosciences, Piscataway, NJ) by vacuum blotting. The membrane was subjected to hybridization with labeled probes using Express Hyb (BD Biosciences clontech, Palo Alto, CA). Probes were obtained by PCR using GetLarge full-length cDNA human placenta library (Genemed, San Francisco, CA) as a template and the following primers:

| | |
|---|---|
| Leptin: | 5'-cttcccactggafft-3' (forward); |
| | 5'-catccctcacctccttcaaa-3' (reverse) |
| Adiponectin: | 5'-cccaggactgggaacatagcat-3' (forward); |
| | 5'-tcagcattcagtgtgggattgg-3' (reverse) |
| GAPDH: | 5'-acagtcagccgcatcttctt-3' (forward); |
| | 5'-gtcttctgggtggcagtgat-3' (reverse). |

Probes were labeled with α-³²P dCTP, using Rediprime kit (Amersham Biosciences, Piscataway, NJ).

### 2. RESULTS

### 2.1 TRIGLYCERIDE ACCUMULATION

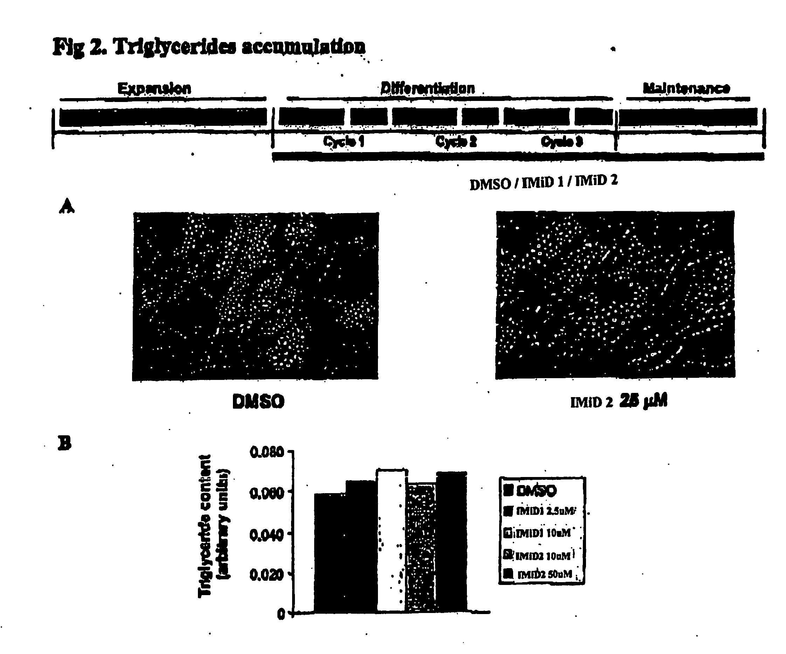

Triglyceride accumulation was observed by microscopy, and quantified using Oil Red O staining. As shown in FIG. 2A, 1-oxo-(2,6-dioxopiperidin-3-yl)-4-aminoisoindoline does not alter the ability of the cells to accumulate triglyceride. Indeed, lipid vacuoles were both present in cells cultured with DMSO and 1-oxo-(2,6-dioxopiperidin-3-yl)-4-aminoisoindoline. Quantification of triglyceride accumulation using Oil Red O staining confirmed this result. As shown in FIG. 2B, no significant difference was observed in triglyceride accumulation in cells cultured with DMSO, 1-oxo-(2,6-dioxopiperidin-3-yl)-4-aminoisoindoline, or 1,3-dioxo-(2,6-dioxopiperidin-3-yl)-4-aminoisoindoline.

### 2.2 LEPTIN SECRETION

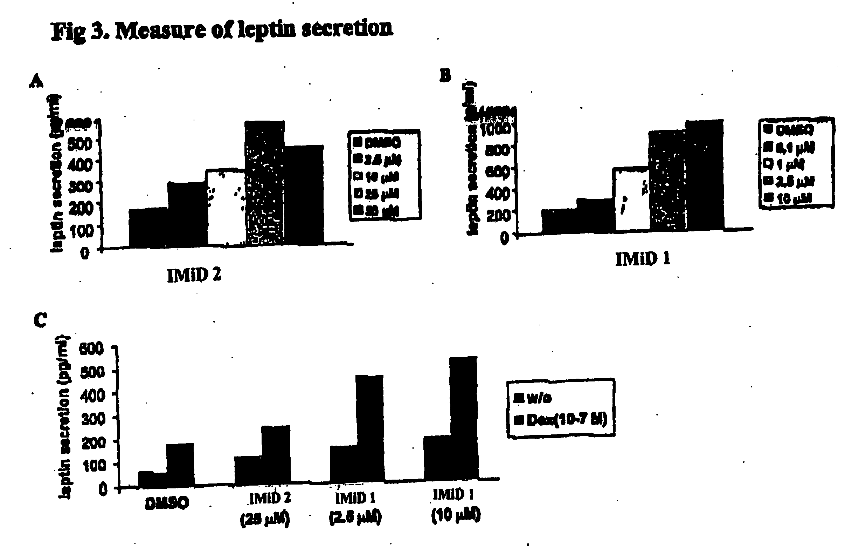

The effects of the immunomodulatory compounds on leptin secretion of adipocytes were examined. As shown in FIG. 3A, adipocytes differentiated in the presence of 1-oxo-(2,6-dioxopiperidin-3-yl)-4-aminoisoindoline, at a concentration of 2.5, 10, 25, or 50 µM, secreted higher levels of leptin than the cells differentiated in DMSO. A dose dependent response was observed, with a maximum at 25 µM.

As shown in FIG. 3B, a similar result was observed for adipocytes differentiated in the presence of 1,3-dioxo-(2,6-dioxopiperidin-3-yl)-4-aminoisoindoline, at a concentration of 0.1, 1, 2.5, or 10 µM. Dose-dependent response was also observed, with a maximum at 10 µM of 1,3-dioxo-(2,6-dioxopiperidin-3-yl)-4-aminoisoindoline.

Furthermore, as shown in FIG. 3C, both 1-oxo-(2,6-dioxopiperidin-3-yl)-4-aminoisoindoline and 1,3-dioxo-(2,6-dioxopiperidin-3-yl)-4-aminoisoindoline enhanced dexamethasone induced leptin secretion, when dexamethasone was added to the cells for 15 hours at the end of differentiation. These results show that the immunomodulatory compounds of the invention enhance leptin secretion in adipocytes.

### 2.3 ADIPONECTIN SECRETION

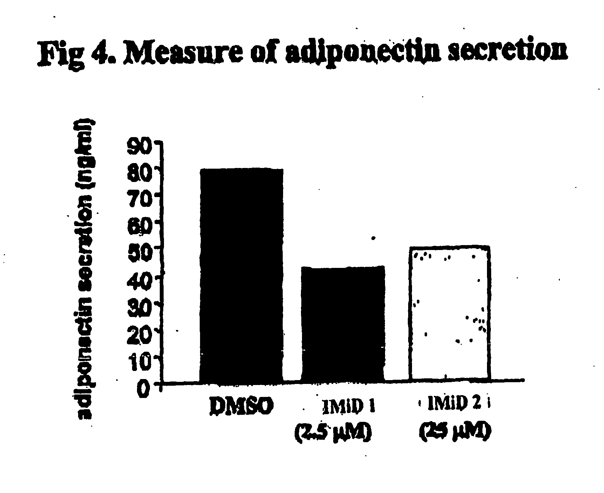

Adiponectin secretion of adipocytes was examined, and the results are shown in FIG. 4. As shown in the figure, adipocytes differentiated in the presence of 1-oxy-(2,6-dioxopiperidin-3-yl)-4-aminoisoindoline or 1,3-dioxo-(2,6-dioxopiperidin-3-yl)-4-aminoisoindoline showed lower levels of adiponectin secretion than the cells differentiated in DMSO. These results show that adiponectin secretion in adipocytes is down-regulated by the immunomodulatory compounds.

### 2.4 DETERMINATION OF mRNA LEVELS

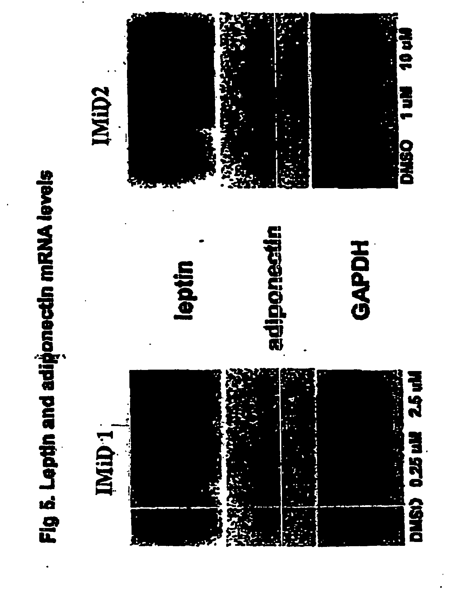

To examine whether the up-regulation of leptin secretion and down-regulation of adiponectin secretion are associated with the change of mRNA levels of these proteins, mRNA levels of leptin and adiponectin were determined using northern blot. As shown in FIG. 5, the presence of 1-oxo-(2,6-dioxopiperidin-3-yl)-4-aminoisoindoline or 1,3-dioxo-(2,6-dioxopiperidin-3-yl)-4-aminoisoindoline dose-dependently increase the level of leptin mRNAs. Conversely, dose-dependent decrease in adiponectin mRNAs was also observed in adipocytes treated with those immunomodulatory compounds. GAPDH mRNA (control) showed no significant change. These results show that the change in leptin and adiponectin secretion is associated with the change in mRNA levels of these proteins.

### 2.5 MSC VIABILITY AND PROLIFERATION

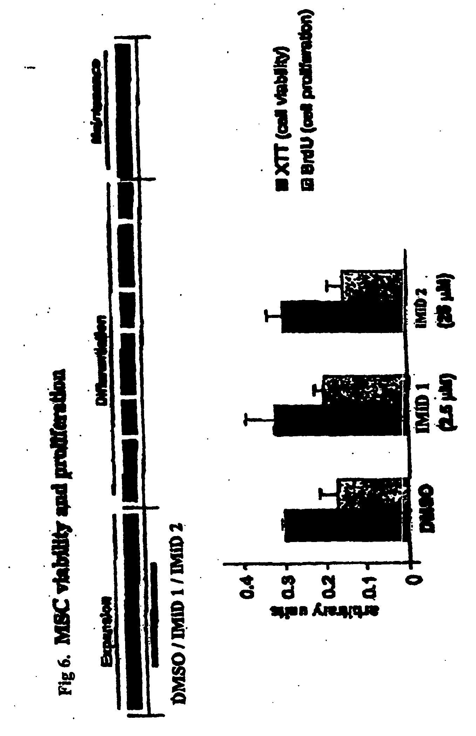

The viability and proliferation of MSCs treated with DMSO or immunomodulatory compounds for 2 days were examined. The viability and proliferation were determined using XTT based calorimetric assay and BrdU incorporation assay, respectively. As shown in Figure 6, neither 1-oxo-(2,6-dioxopiperidin-3-yl)-4-aminoisoindoline nor 1,3-dioxo-(2,6-dioxopiperidin-3-yl)-4-aminoisoindoline caused significant change in cell viability and proliferation. These results suggest that the increase in leptin secretion caused by the immunomodulatory compounds is not due to the regulation of cell viability or number by these compounds.

## Claims

1. An immunomodulatory compound, or a pharmaceutically acceptable salt, solvate, or stereoisomer thereof for use in treating, managing or preventing a disorder associated with a hypoleptinemic state, or a symptom thereof;
wherein the disorder is a metabolic or an eating disorder, hypoleptinemia related neuroendocrine dysfunction, hypoleptinemia related immunodeficiency, hypothalamic amenorrhea, acromegaly, hypoleptinemia related infertility syndrome, skin damage, wound, long-term hemodialysis, or loss of hair; and
wherein the immunomodulatory compound is of
(a) formula (I): 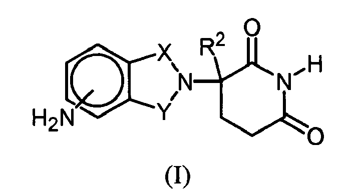 wherein one of X and Y is C=O, the other of X and Y is C=O or CH₂, and R² is hydrogen or methyl; or
(b) formula (II): 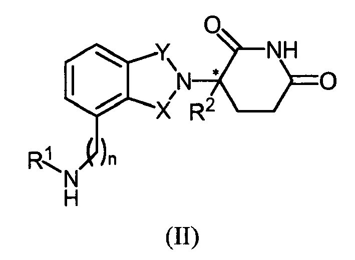 wherein
one of X and Y is C=O and the other is CH₂ or C=O;
R¹ is H, (C₁-C₈)alkyl, (C₃-C₇)cycloalkyl, (C₂-C₈)aLkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)hetemcycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)atkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵, or the
R⁶ groups join to form a heterocycloalkyl group;
n is 0 or 1; and
* represents a chiral-carbon center.

2. The compound for use of claim 1, which is 4-(amino)-2-(2,6-dioxo(3-piperidyl))-isoindoline-1,3-dione or 3-(4-amino-1-oxo-1,3-dihydro-isoindol-2-yl)-piperidine-2,6-dione.

3. The compound for use of claim 1 or 2, which is enantiomerically pure.

4. The compound for use of any of claims 1 to 3, wherein the metabolic or eating disorder is lipodystrophy syndrome or anorexia nervosa.

5. The compound for use of any of claims 1 to 4, wherein the symptom is neuroendocrine defect, immunodeficiency, high glucose and triglyceride levels, enlarged liver, amenorrhea, delayed puberty, hypothyroidism, hypercorticosolism, abnormal bone metabolism, or alteration in growth hormone axis.

6. The compound for use of any of claims 1 to 5, wherein the compound, or a pharmaceutically acceptable salt, solvate, or stereoisomer thereof, is formulated to be administered in combination with a therapeutically or prophylactically effective amount of a second active agent.

7. The compound for use of claim 6, wherein the second active agent is an antibiotic, a thiazolidinedione, dexamethasone, insulin, a hypoglycemic agent, a lipid lowering agent, fluoxetine, clomiphene citrate, a gonadotropins, GnRH, a corticosteroid, minoxidil, finasteride, amylin, pramlintide, or clomipramine.

8. The compound for use of claim 7, wherein the antibiotic is chloramphenicol, tetracycline, chlortetracycline, sulfasalazine, ampicillin, penicillin, or trimethoprim-sulfamethoxazole.

9. The compound for use of claim 7, wherein the hypoglycemic agent is metformin or TZD (thiazolidinedione).

10. The compound for use of claim 7, wherein the lipid lowering agent is a fibrate or a statin.

11. The compound for use of claim 7, wherein the corticosteroid is betamethasone, clobetasol, amcinonide, desoximethasone, diflorasone, fluocinonide, halcinonide, mometasone, fluticasone, triamcinolone, fluocinolone, flurandrenolide, hydrocortisone, alclometasone, desonide, flumethasone, or pramoxine.

12. The compound for use of claim 7, wherein the second active agent is dexamethasone.

## Patentansprüche

1. Immunmodulatorische Verbindung oder ein pharmazeutisch verträgliches Salz, Solvat oder Stereoisomer davon zur Verwendung beim Behandeln, Regeln oder Vorbeugen einer Störung, die mit einem hypoleptinämischen Zustand in Zusammenhang steht, oder eines Symptoms davon;
wobei die Störung eine Stoffwechsel- oder eine Essstörung, mit Hypoleptinämie in Zusammenhang stehende neuroendokrine Dysfunktion, mit Hypoleptinämie in Zusammenhang stehende Immunschwäche, hypothalamische Amenorrhö, Akromegalie, mit Hypoleptinämie in Zusammenhang stehendes Unfruchtbarkeitssyndrom, Hautschaden, Wunde, Langzeithämodialyse oder Haarausfall ist; und
wobei die immunmodulatorische Verbindung
(a) Formel (I): 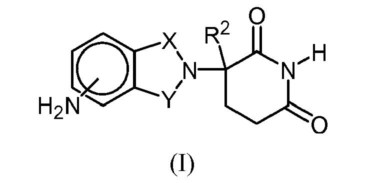 wobei eines von X und Y C=O ist, das andere von X und Y C=O oder CH₂ ist, und R² Wasserstoff oder Methyl ist; oder
(b) Formel (II): 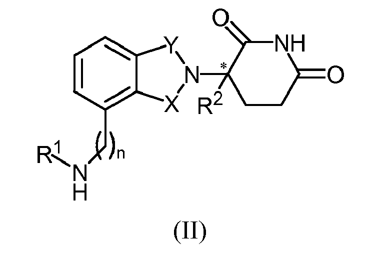 wobei
eines von X und Y C=O ist und das andere CH₂ oder C=O ist;
R¹ H, (C₁-C₈)Alkyl, (C₃-C₇)Cycloalkyl, (C₂-C₈)Alkenyl, (C₂-C₈)Alkinyl, Benzyl, Aryl, (C₀-C₄)Alkyl-(C₁-C₆)heterocycloalkyl, (C₀-C₄)Alkyl-(C₂-C₅)heteroaryl, C(O)R³, C(S)R³, C(O)OR⁴, (C₁-C₈)Alkyl-N(R⁶)₂, (C₁-C₈)Alkyl-OR⁵, (C₁-C₈)Alkyl-C(O)OR⁵, C(O)NHR³, C(S)NHR³, C(O)NR³R^{3'}, C(S)NR³R^{3'} oder (C₁-C₈)Alkyl-O(CO)R⁵ ist;
R² H, F, Benzyl, (C₁-C₈)Alkyl, (C₂-C₈)Alkenyl oder (C₂-C₈)Alkinyl ist;
R³ und R^{3'} unabhängig (C₁-C₈)Alkyl, (C₃-C₇)Cycloalkyl, (C₂-C₈)Alkenyl, (C₂-C₈)Alkinyl, Benzyl, Aryl, (C₀-C₄)Alkyl-(C₁-C₆)heterocycloalkyl, (Co-C₄)Alkyl-(C₂-C₅)heteroaryl, (C₀-C₈)Alkyl-N(R⁶)₂, (C₁-C₈)Alkyl-OR⁵, (C₁-C₈)Alkyl-C(O)OR⁵, (C₁-C₈)Alkyl-O(CO)R⁵ oder C(O)OR⁵ sind;
R⁴ (C₁-C₈)Alkyl, (C₂-C₈)Alkenyl, (C₂-C₈)Alkinyl, (C₁-C₄)Alkyl-OR⁵, Benzyl, Aryl, (C₀-C₄)Alkyl-(C₁-C₆)heterocycloalkyl oder (C₀-C₄)Alkyl-(C₂-C₅)heteroaryl ist;
R⁵ (C₁-C₈)Alkyl, (C₂-C₈)Alkenyl, (C₂-C₈)Alkinyl, Benzyl, Aryl oder (C₂-C₅)Heteroaryl ist;
jedes Vorkommen von R⁶ unabhängig H, (C₁-C₈)Alkyl, (C₂-C₈)Alkenyl, (C₂-C₈)Alkinyl, Benzyl, Aryl, (C₂-C₅)Heteroaryl oder (C₀-C₈)Alkyl-C(O)O-R⁵ ist oder die Reste R⁶ verbunden sind, um einen Heterocycloalkylrest zu bilden;
n 0 oder 1 ist; und
* ein chirales Kohlenstoffzentrum darstellt;
aufweist.

2. Verbindung zur Verwendung nach Anspruch 1, die 4-(Amino)-2-(2,6-dioxo(3-piperidyl))-isoindolin-1,3-dion oder 3-(4-Amino-1-oxo-1,3-dihydro-isoindol-2-yl)-piperidine-2,6-dion ist.

3. Verbindung zur Verwendung nach Anspruch 1 oder 2, die enantiomerenrein ist.

4. Verbindung zur Verwendung nach einem der Ansprüche 1 bis 3, wobei die Stoffwechsel- oder Essstörung Lipodystrophie-Syndrom oder Anorexia Nervosa ist.

5. Verbindung zur Verwendung nach einem der Ansprüche 1 bis 4, wobei das Symptom neuroendokriner Defekt, Immunschwäche, hohe Glucose- und Triglyceridspiegel, vergrößerte Leber, Amenorrhö, verspätete Pubertät, Hypothyrodismus, Hypercorticosolismus, abnormaler Knochenstoffwechsel oder Änderung der Wachstumshormonachse ist.

6. Verbindung zur Verwendung nach einem der Ansprüche 1 bis 5, wobei die Verbindung oder ein pharmazeutisch verträgliches Salz, Solvat oder Stereoisomer davon hergerichtet ist, in Kombination mit einer therapeutisch oder prophylaktisch wirksamen Menge eines zweiten Wirkstoffs verabreicht zu werden.

7. Verbindung zur Verwendung nach Anspruch 6, wobei der zweite Wirkstoff ein Antibiotikum, ein Thiazolidindion, Dexamethason, Insulin, ein hypoglykämisches Mittel, ein lipidsenkendes Mittel, Fluoxetin, Clomiphencitrat, ein Gonadotropin, GnRH, ein Corticosteroid, Minoxidil, Finasterid, Amylin, Pramlintid oder Clomipramin ist.

8. Verbindung zur Verwendung nach Anspruch 7, wobei das Antibiotikum Chloramphenicol, Tetracyclin, Chlortetracyclin, Sulfasalazin, Ampicillin, Penicillin, oder Trimethoprim-Sulfamethoxazol ist.

9. Verbindung zur Verwendung nach Anspruch 7, wobei das hypoglykämische Mittel Metformin oder TZD (Thiazolidindion) ist.

10. Verbindung zur Verwendung nach Anspruch 7, wobei das lipidsenkende Mittel ein Fibrat oder ein Statin ist.

11. Verbindung zur Verwendung nach Anspruch 7, wobei das Corticosteroid Betamethason, Clobetasol, Amcinonid, Desoximethason, Diflorason, Fluocinonid, Halcinonid, Mometason, Fluticason, Triamcinolon, Fluocinolon, Flurandrenolid, Hydrocortison, Alclometason, Desonid, Flumethason oder Pramoxin ist.

12. Verbindung zur Verwendung nach Anspruch 7, wobei der zweite Wirkstoff Dexamethason ist.

## Revendications

1. Composé immunomodulateur ou l'un de ses sels, solvates ou stéréoisomères acceptables au plan pharmaceutique, pour une utilisation dans le traitement, la gestion ou la prévention d'un trouble associé à un état d'hypoleptinémie, ou à l'un de ses symptômes ;
dans lequel le trouble est un trouble métabolique ou un trouble de l'alimentation, un disfonctionnement neuro-endocrinien associé à une hypoleptinémie, une immunodéficience associée à une hypoleptinémie, une aménorrhée hypothalamique, une acromégalie, un syndrome d'infertilité associé à une hypoleptinémie, des lésions cutanées, une blessure, une hémodialyse de longue durée ou la perte de cheveux ; et
dans lequel le composé immunomodulateur est représenté par
(a) la formule (I) : 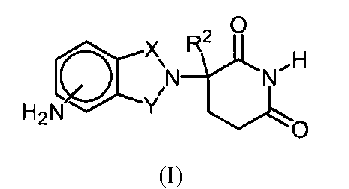 dans laquelle l'un ou l'autre des résidus X et Y représente C=O, l'autre résidu X ou Y représentant C=O ou CH₂, et R² est un atome d'hydrogène ou un méthyle ; ou
(b) la formule (II) : 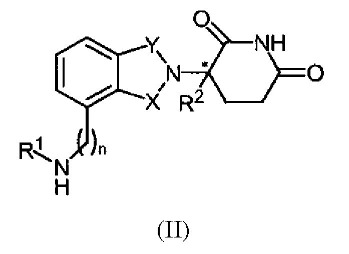
dans laquelle l'un ou l'autre des résidus X et Y représente C=O et l'autre représente CH₂ ou C=O ;
R¹ représente H, un alkyle C₁-C₈, un cycloalkyle C₃-C₇, un alcényle C₂-C₈, un alcynyle C₂-C₈, un benzyle, un aryle, un alkyle(C₀-C₄)-hétérocycloalkyle(C₁-C₆), un alkyle(C₀-C₄)-hétéroaryle(C₂-C₅), un groupe C(O)R³, C(S)R³, C(O)OR⁴, un alkyle(C₁-C₈)-N(R⁶)₂, un alkyle(C₁-C₈)-OR⁵, un alkyle(C₁-C₈)-C(O)OR⁵, un groupe C(O)NHR³, C(S)NHR³, C(O)NR³R³', C(S)NR³R³' ou un alkyle(C₁-C₈)-O(CO)R⁵ ;
R² représente l'élément H, F, un benzyle, un alkyle C₁-C₈, un alcényle C₂-C₈ ou un alcynyle C₂-C₈ ;
R³ et R³' représentent indépendamment un alkyle C₁-C₈, un cycloalkyle C₃-C₇, un alcényle C₂-C₈, un alcynyle C₂-C₈, un benzyle, un aryle, un alkyle(C₀-C₄)-hétérocycloalkyle(C₁-C₆), un alkyle(C₀-C₄)-hétéroaryle(C₂-C₅), un alkyle(C₀-C₈)-N(R⁶)₂, un alkyle(C₁-C₈)-OR⁵, un alkyle(C₁ C₈)-C(O)OR⁵, un alkyle(C₁-C₈)-O(CO)R⁵ ou un groupe C(O)OR⁵ ;
R⁴ représente un alkyle C₁-C₈, un alcényle C₂-C₈, un alcynyle C₂-C₈, un alkyle(C₁-C₄)-OR⁵, un benzyle, un aryle, un alkyle(C₀-C₄)-hétérocycloalkyle(C₁-C₆) ou un alkyle(C₀-C₄)-hétéroaryle(C₂-C₅) ;
R⁵ représente un alkyle C₁-C₈, un alcényle C₂-C₈, un alcynyle C₂-C₈, un benzyle, un aryle ou un hétéroaryle C₂-C₅ ;
chaque R⁶ présent représente indépendamment l'élément H, un alkyle C₁-C₈, un alcényle C₂-C₈, un alcynyle C₂-C₈, un benzyle, un aryle, un hétéroaryle C₂-C₅ ou un alkyle(C₀-C₈)-C(O)O-R⁵, ou les groupes R⁶ sont réunis de manière à former un groupe hétérocycloalkyle ;
n vaut 0 ou 1 ; et
* représente un centre de carbone chiral.

2. Composé pour une utilisation selon la revendication 1, lequel composé est la 4-(amino)-2-(2,6-dioxo(3-pipéridyl))-isoindoline-1,3-dione ou la 3-(4-amino-1-oxo-1,3-dihydro-isoindol-2-yl)-pipéridine-2,6-dione.

3. Composé pour une utilisation selon les revendications 1 ou 2, qui présente un énantiomère pur.

4. Composé pour une utilisation selon les revendications 1 à 3, dans lequel le trouble métabolique ou le trouble de l'alimentation est un syndrome de lipodystrophie ou une anorexie mentale.

5. Composé pour une utilisation selon l'une quelconque des revendications 1 à 4, dans lequel le symptôme est une anomalie neuroendocrinienne, une immunodéficience, des niveaux élevés de glucose et de triglycérides, une hépatomégalie, une aménorrhée, un retard pubertaire, une hypothyroïdie, un hypercortisolisme, une anomalie du métabolisme osseux ou une altération de l'axe de l'hormone de croissance.

6. Composé pour une utilisation selon l'une quelconque des revendications 1 à 5, dans lequel le composé ou l'un de ses sels, solvates ou stéréoisomères acceptables au plan pharmaceutique, est formulé pour être administré Combinaison avec une quantité efficace au plan thérapeutique ou prophylactique d'un second agent actif.

7. Composé pour une utilisation selon la revendication 6, dans lequel le second agent actif est un antibiotique, une thiazolidinedione, la dexaméthasone, l'insuline, un agent hypoglycémique, un agent hypolipidémiant, la fluoxétine, le citrate de clomiphène, des gonadotrophines, la GnRH, un corticostéroïde, le minoxidil, le finastéride, l'amyline, le pramlintide ou la clomipramine.

8. Composé pour une utilisation selon la revendication 7, dans lequel l'antibiotique est le chloramphénicol, la tétracycline, la chlortétracycline, la sulfasalazine, l'ampicilline, la pénicilline ou le triméthoprim-sulfaméthoxazole.

9. Composé pour une utilisation selon la revendication 7, dans lequel l'agent hypoglycémique est la metformine ou le TZD (thiazolidinedione).

10. Composé pour une utilisation selon la revendication 7, dans lequel l'agent hypolipidémiant est un fibrate ou une statine.

11. Composé pour une utilisation selon la revendication 7, dans lequel le corticostéroïde est la bétaméthasone, le clobétasol, l'amcinonide, la désoximéthasone, la diflorasone, le fluocinonide, l'halcinonide, la mométasone, la fluticasone, la triamcinolone, la fluocinolone, le flurandrénolide, l'hydrocortisone, l'alclométasone, le désonide, la fluméthasone ou la pramoxine.

12. Composé pour une utilisation selon la revendication 7, dans lequel le second agent actif est la dexaméthasone.
